# EUROPEAN PATENT APPLICATION

(11) **EP 4 147 695 A1**
(43) Date of publication of application: **15.03.2023**
(21) Application number: 21800172.5
(22) Date of filing: 07.05.2021
(51) Int. Cl.: A61K 31/42, A61K 31/522, A61P 25/00, A61P 25/28, A61P 25/24

(54) **CYCLOSERINE AND PENTOXIFYLLINE COMBINATION THERAPY FOR TREATMENT OF DEPRESSION**

(30) Priority: 07.05.2020 KR 20200054618
(71) Applicant: Neurorive Inc, Seoul 02455 (KR)
(72) Inventor: LEE, Sukchan, Yongin-si Gyeonggi-do 16823 (KR); CHI, Yong Ha, Seogwipo-si Jeju-do 63560 (KR); JANG, Dong Cheol, Wonju-si Gangwon-do 26469 (KR); HWANG, Hyeonjoo, Seoul 07777 (KR)
(74) Representative: Mewburn Ellis LLP
(86) International application number: PCT/KR2021/005711
(87) International publication number: WO 2021/225397

(57) **Abstract**

The present invention provides to a composite composition for preventing or treating depression comprising (i) cycloserine or a pharmaceutically acceptable salt thereof; and (ii) pentoxifylline or a pharmaceutically acceptable salt thereof. The composite composition may be effectively used as an antidepressant.

## Description

### [Technical Field]

The present invention relates to a combination therapy of cycloserine and pentoxifylline for the treatment of depression.

### [Background Art]

Stress refers to a detrimental response or change in an organism caused by an external stimulus. In general, when the stimulus on the living body becomes more than a certain degree, it acts harmfully. At this time, the living body causes tension headache, migraine, hypertension, dyspepsia, fatigue, pain, hair loss, and rough skin in response to a certain threat regardless of the type of stimulus, if it persists chronically, nonspecific general adaptice syndrome, such as causing various neuroses and gastric ulcer, is defined as stress (Selye, 1958, Longmans Green and Co.). According to several recent reports, it is known that more than 90% of diseases are caused by stress. Recently, the Ministry of Health and Welfare of Korea also classifies stress as a new disease code. Specifically, it has been reported that various diseases including mental disorders such as depression, digestive disorders such as gastraneuria and duodenal ulcer, hormonal disorders such as stress hyperthyroidism, skin disorders such as stress melasma or acne, and cardiovascular disorders such as arrhythmias or angina pectoris are caused by stress.

Depression is an emotional pathological phenomenon that occurs regardless of the objective situation. All of the patients' lives are covered with depressed moods, decreased interest, and anhedonia, and become obsessed with deprivation of mental exercise, a feeling of pessimism, and despair. It is a disease that leads to suicide attempts because suicide is motivated, and is manifested by a variety of physical symptoms such as decreased appetite, insomnia, constipation, loss of libido, and the like. The depression has recently emerged as a serious social problem.

Most of the current antidepressants have pharmacological actions that increase the concentration of neurotransmitters in central serotonin or noradrenaline synapses. The antidepressants that are widely used may be classified into tricyclic antidepressants (TCA), monoamine oxidase inhibitors (MAOI), selective serotonin reuptake inhibitors (SSRI), and the like, depending on mechanisms that increase the concentration of neurotransmitters. Even though these conventional antidepressants are relatively effective, in some cases, severe side effects and effects that do not meet expectations may appear. There are a number of patients who do not respond well to antidepressant drugs (Thase et al. 2001), have a high recurrence rate (Frank et al. 1991), and are not able to take the drug due to severe side effects (Gumnick and Nemeroff 2000). TCA such as imipramine, desipramine, and the like, have severe side effects such as hypotension, heart dysfunction, and the like. The most widely used SSRI such as fluoxetine, sertraline, and the like, cause nausea, gastrointestinal bleeding, sexual dysfunction, or the like.

D-cycloserine (DCS) is an antibiotic substance approved under the brand name Seromycin, and is known to be a partial agonist for NMDA receptors with regard to the nervous system. Seromycin capsules, which are cycloserine used as a medicament, are administered orally twice a day. The drug concentration thereof is determined in consideration of body weight, medical condition, seromycin serum level, and the like, and should not exceed 1,000 mg per day.

On the other hand, pentoxifylline has been known for circulatory diseases such as peripheral vascular disease, blood viscosity lowering and blood flow improvement, antithrombotic effects, and the like, and it can be used for cerebral circulation disorders in relation to the central nervous system. Pentoxifylline is administered orally 2-3 times a day, and about 400 mg can be administered at a time.

It is known that cycloserine and pentoxifylline are able to act on the central nervous system through different mechanisms, respectively, but there is no known case of using the above drugs in combination to treat depression.

### [Disclosure]

### [Technical Problem]

An object of the present invention is to provide a use for preventing or treating depression according to the combined administration of cycloserine and pentoxifylline.

Another object of the present invention is to provide a use for improving depression according to the combined administration of cycloserine and pentoxifylline.

### [Technical Solution]

In the present invention, the inventors confirmed that when cycloserine and pentoxifylline were administered in combination, an antidepressant effect was significantly improved compared to each single administration.

In one aspect, the present invention provides a pharmaceutical composition for preventing or treating depression comprising: (i) cycloserine or a pharmaceutically acceptable salt thereof; and (ii) pentoxifylline or a pharmaceutically acceptable salt thereof as active ingredients.

In the pharmaceutical composition, the cycloserine may be D-cycloserine.

In the pharmaceutical composition, a weight ratio of (i) the cycloserine or the pharmaceutically acceptable salt thereof; and (ii) the pentoxifylline or the pharmaceutically acceptable salt thereof may be 1 : 1 to 1 : 12, and specifically, 1 : 2 to 1 : 8.

The pharmaceutical composition may be administered orally, and each active ingredient may be administered simultaneously or at different times. For example, the pharmaceutical composition may be a combination preparation in which each active ingredient is administered simultaneously.

In still aspect, the present invention provides a food composition for improving depression comprising: (i) cycloserine or a pharmaceutically acceptable salt thereof; and (ii) pentoxifylline or a pharmaceutically acceptable salt thereof.

### [Advantageous Effects]

The combined administration of cycloserine and pentoxifylline according to the present invention has an excellent effect of reducing depression compared to each single administration, thereby being effectively used as an antidepressant.

### [Description of Drawing]

FIG. 1 shows results of a forced swimming test (FST) in mouse depending on a combination treatment of D-cycloserine and pentoxifylline.
FIG. 2 shows results of a tail suspension test (TST) in mouse depending on a combination treatment of D-cycloserine and pentoxifylline.

### [Best Mode]

Hereinafter, the present invention will be described in detail as exemplary embodiments of the present invention with reference to the accompanying drawings. However, the following exemplary embodiments are presented as examples of the present invention, and in a case where it is determined that detailed descriptions of a technique or configuration well known to those skilled in the art may unnecessarily obscure the gist of the present invention, detailed descriptions thereof may be omitted, and thus the present invention is not limited thereto. The present invention may be variously modified and applied within the description of the claims to be described below and within the equivalent scope interpreted therefrom.

In addition, terminologies used in the present specification are terms used to appropriately express preferred embodiments of the present invention, which may vary depending on the intention of users or operators, or customs in the field to which the present invention belongs, and the like. Accordingly, definitions of these terms should be established based on the contents throughout the present specification. Throughout the specification, when a part "comprise" a component, it means that the part may further include other components rather than excluding the other components unless otherwise specified.

All technical terms used in the present invention, unless otherwise defined, are used in the same meaning as those of ordinary skill in the art generally understand in the related field of the present invention. In addition, preferred methods or samples are described in the present specification, but those similar thereto or the equivalents thereof are included in the scope of the present invention. Contents of all publications described in the present specification by reference are incorporated into the present invention.

The inventors confirmed that the combined administration of cycloserine and pentoxifylline had a remarkably excellent effect of antidepressant compared to each single administration, and completed the present invention.

In one aspect, the present invention provides a pharmaceutical composition for preventing or treating depression comprising: (i) cycloserine or a pharmaceutically acceptable salt thereof; and (ii) pentoxifylline or a pharmaceutically acceptable salt thereof.

In the pharmaceutical composition of the present invention, the cycloserine may be D-cycloserine represented by the following Formula 1:

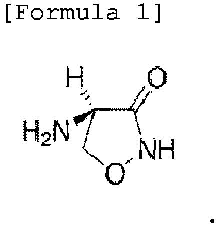

On the other hand, pentoxifylline is a non-selective PDE inhibitor belonging to xanthine derivative, and is mechanistically distinguished from a drug belonging to a competitive PDE inhibitor, for example, PDE1 inhibitor (e.g. vinpocetine), PDE2 inhibitor (e.g. EHNA(erythro-9-(2-hydroxy-3-nonyl)adenine), BAY 60-7550(2-[(3,4-dimetoxyphenyl)methyl]-7-[(1R)-1-hydroxyethyl]-4-phenylbutyl)-5-methyl-imidazo[5,1-f][1,2,4]triazine-4(1H)-on), oxindole, PDP(9-(6-phenyl-2-oxohex-3-yl)-2-(3,4-dimethoxybenzyl)-purine-6-on))), PDE3 inhibitor (e.g. anagrelide, cilostazol, enoximone, inamrinone, milinone, pimobendan), PDE4 inhibitor (e.g. apremilast, drotaverine, ibudilast, luteolin, mesembrine, piclamilast, roflumilast, rolipram), PDE5 inhibitor (e.g. avanafil, dipyridamole, icariin, sildenafil, tadalafil, udenafil, vardenafil), PDE6 inhibitor, PDE7 inhibitor (e.g. quinazoline), PDE8 inhibitor, PDE9 inhibitor, PDE10 inhibitor (e.g. papaverine), PDE11 inhibitor, PDE12 inhibitor or combination thereof.

In addition, pentoxifylline is distinguished from other xanthine derivatives belonging to non-selective PDE inhibitors, such as caffeine, paraxanthine, theobromine and theophylline.

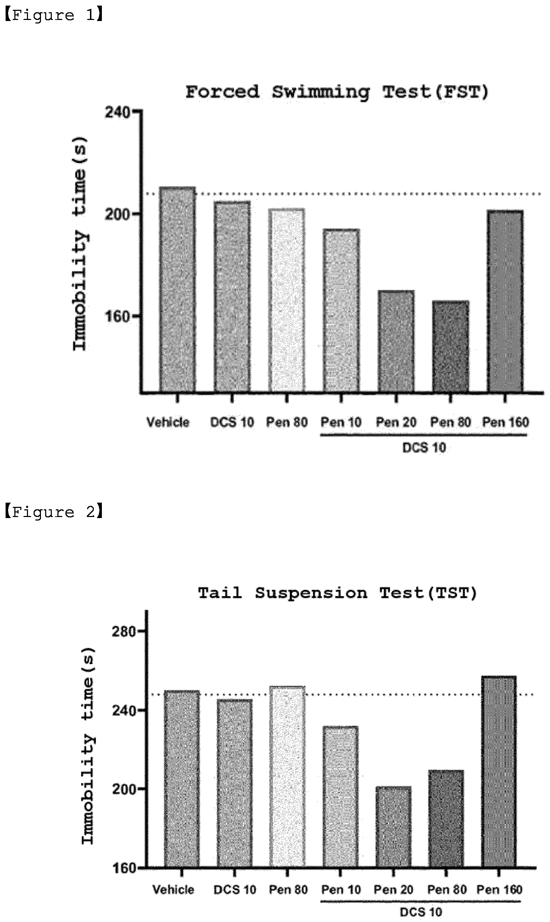

In an exemplary embodiment of the present invention, it was confirmed that the combined administration of D-cycloserine and pentoxifylline showed effective in treating depression (FIGS. 1 and 2).

In the present invention, the pharmaceutically acceptable salt refers to a salt commonly used in the pharmaceutical industry, and for example, may include salts of inorganic ions such as sodium, potassium, calcium, magnesium, lithium, copper, manganese, zinc, iron, and the like, salts of inorganic acids such as hydrochloric acid, phosphoric acid, sulfuric acid, and the like, and in addition thereto, may include salts of organic acids such as ascorbic acid, citric acid, tartaric acid, lactic acid, maleic acid, malonic acid, fumaric acid, glycolic acid, succinic acid, propionic acid, acetic acid, orotic acid, gluconic acid, acetylsalicylic acid, and the like, and amino acid salts such as lysine, arginine, guanidine, and the like. In addition, the pharmaceutically acceptable salt may include salts of organic ions such as tetramethyl ammonium, tetraethyl ammonium, tetrapropyl ammonium, tetrabutyl ammonium, benzyl trimethyl ammonium, benzethonium, and the like, that may be used in pharmaceutical reactions, purification and separation processes. However, types of salts meant in the present invention are not limited to salts that are listed above.

In the present invention, by performing the forced swimming test (FST) and the tail suspension test (TST), which are antidepressant efficacy tests targeting animal models, the immobility reduction effect of the cycloserine and pentoxifylline combination composition was confirmed. Therefore, the combined composition of the present invention may be usefully used as a composition for preventing or treating depression. In addition, from the results of the FST and TST tests, the combined composition of the present invention is expected to be usefully utilized as a therapeutic agent for anxiety disorders (for example, panic disorder, obsessive compulsive disorder, post traumatic stress disorder, acute stress disorder, hypochondriasis, dissociative disorder).

In an exemplary embodiment of the present invention, it was confirmed that the combined administration of cycloserine and pentoxifylline showed remarkably excellent results in behavioral tests such as a forced swimming test (FST), tail suspension test (TST), and the like, in experimental animals (FIGS. 1 and 2) . Therefore, the pharmaceutical composition of the present invention may be effectively utilized for preventing or treating depression or anxiety disorder.

In the pharmaceutical composition of the present invention, a weight ratio of (i) the cycloserine or the pharmaceutically acceptable salt thereof; and (ii) the pentoxifylline or the pharmaceutically acceptable salt thereof may be 1 : 1 to 1 : 12, and more specifically, 1 : 2 to 1 : 8. For example, the pharmaceutical composition may comprise 50 to 250 mg of the cycloserine and 100 to 600 mg of the pentoxifylline, respectively, in the preparation, but is not limited thereto. In one embodiment, the pharmaceutical composition may comprise 100 to 400 mg of pentoxifylline per about 50 mg of D-cycloserine in the preparation.

The pharmaceutical composition of the present invention may be administered in a therapeutically effective amount. The therapeutically effective amount refers to a drug dosage that exerts an effective antidepressant effect. The suitable total daily dosage may be determined by the treating physician within proper medical judgment range. It is preferable to apply differently the specific therapeutically effective amount for a specific patient depending on various factors including the type and degree of the reaction to be achieved, the type and amount of drugs to be administered in combination, a specific composition used whether other preparations are used in some cases, the patient's age, weight, general health status, sex and diet, time of administration, route of administration, duration of treatment, and similar factors well known in the medical field.

The pharmaceutical composition of the present invention may be administered in divided doses once to several times a day. For example, cycloserine or a pharmaceutically acceptable salt thereof and pentoxifylline or a pharmaceutically acceptable salt thereof may be administered based on a mouse at a dose of about 10 to 120 mg/kg, more specifically about 20 to 80 mg/kg of pentoxifylline (Pen) per dose of about 10 mg/kg of DCS, it was confirmed that the results of the forced swimming test (FST) and the tail suspension test (TST) were significantly improved at the above-described DCS : Pen weight ratio in the dose of the range (Figs. 1 and 2).

Therefore, it is possible to convert the preferred dose in human in consideration of the optimal dose to be administered to mouse confirmed in the present invention, the mouse-human dose-response relationship, and no-observed-adverse-effect level (NOAEL), and the like. The dose conversion factor may be expressed by using the human equivalent dose (HED) calculation method described in the document (see, FDA, US. "Guidance for Industry, Estimating the maximum safe starting dose in initial clinical trials for therapeutics in adult healthy volunteers." FDA, ed (2005)). The mouse-human dose conversion factor suggested in the above document is 12.3, and a dose of A/12.3 (mg/kg) is derived by dividing a dose (A) of the drug identified in the mouse by the conversion factor. In general, one tablet is made for an adult weighing 60 kg. If the value of A/12.3 (mg/kg) is multiplied by 60 kg, the dose of about 5 × A (mg), which is the human dose, is derived. Therefore, with reference to the optimal mouse dose ratio of D-cycloserine and pentoxifylline identified in the examples of the present invention, it is possible to derive a preferred drug dose to be administered to humans. In one exemplary embodiment, the combined composition of the present invention may comprise 50 to 250 mg of D-cycloserine and 100 to 600 mg of pentoxifylline, respectively. For example, the D-cycloserine and the pentoxifylline in a combination preparation may be 50 to 100 mg and 100 to 400 mg, respectively.

The pharmaceutical composition of the present invention may be administered orally or parenterally, and is preferably administered orally. In addition, the pharmaceutical composition of the present invention may be used in combination with one or more central nervous system drugs. For example, the pharmaceutical composition may be used in combination with additional pharmaceutical antidepressants, herbal antidepressants, anticonvulsants, mood stabilizers, antipsychotics, benzodiazepines, or a combination thereof for effective treatment of depression.

The pharmaceutical composition of the present invention may include a pharmaceutically acceptable carrier, excipient, and/or diluent for administration. Examples of the carrier, excipient and/or diluent may include, but are not limited to, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oils.

The pharmaceutical composition of the present invention may be prepared in a pharmaceutical formulation using a method well known in the art. In the preparation of the formulation, the active ingredient may be mixed or diluted with a carrier, or encapsulated in a carrier in the form of a container. When the pharmaceutical composition of the present invention is prepared in a formulation for oral administration, for example, the pharmaceutical composition may be formulated as tablets, troches, lozenges, water-soluble or oily suspensions, powders or granules, emulsions, hard or soft capsules, syrups or elixirs.

In one aspect, the present invention provides a method for preventing or treating depression comprising administering to a patient a therapeutically effective amount of (i) cycloserine or a pharmaceutically acceptable salt thereof; and (ii) pentoxifylline or a pharmaceutically acceptable salt thereof. In another aspect, the present invention provides a use of (i) cycloserine or a pharmaceutically acceptable salt thereof; and (ii) pentoxifylline or a pharmaceutically acceptable salt thereof in preparation of antidepressants. The cycloserine, pentoxifylline, and depressive disorders are the same as described above.

In one aspect, the present invention provides a combination for preventing or treating depression comprising: (i) a first preparation including cycloserine or a pharmaceutically acceptable salt thereof; and (ii) a second preparation including pentoxifylline or a pharmaceutically acceptable salt thereof.

In the present invention, the term "combination" refers to a combination of two or more active substances in a formulation and a combination in the sense of individual formulations of active substances that are administered at specified intervals from each other in treatment. Thus, the term "combination", when described in connection with the present invention, includes the clinical realization of co-administration of two or more therapeutically effective compounds.

In the combination of the present invention, the cycloserine may be D-cycloserine.

In the combination of the present invention, the first preparation may comprise 50 to 250 mg of cycloserine or a pharmaceutically acceptable salt thereof, and the second preparation may comprise 100 to 600 mg of pentoxifylline or a pharmaceutically acceptable salt thereof. More specifically, the first preparation may comprise 50 to 100 mg of cycloserine or a pharmaceutically acceptable salt thereof, and 100 to 400 mg of pentoxifylline.

In the combination of the present invention, the first preparation and/or the second preparation may be administered parenterally or orally, respectively, and preferably may be administered orally.

In the combination of the present invention, the first preparation and the second preparation may be administered simultaneously or at different times.

The composition of the present invention may be a pharmaceutical composition or a food composition. When the composition is used as the food composition, cycloserine and pentoxifylline or a pharmaceutically acceptable salt thereof may be added as it is or may be used together with other foods or food ingredients, and may be appropriately used according to a conventional method. The composition may include food supplementary additives acceptable for food in addition to an active ingredient, and an amount of the active ingredient mixed may be appropriately determined according to the purpose of use (prevention, health or therapeutic treatment).

The food composition of the present invention may comprise a health functional food. The term "health functional food" used in the present invention refers to food manufactured and processed in the form of tablets, capsules, powders, granules, liquids pills, and the like, using raw materials or ingredients that have useful functionality for the human body. Here, the term "functionality" refers to obtaining useful effects for health use such as controlling nutrients, physiological action, or the like, on the structure and function of the human body.

In addition, there is no limitation on the type of health food that the composition of the present invention can be used. Further, the composition comprising cycloserine and/or pentoxifylline as an active ingredient of the present invention may be prepared by mixing other suitable adjuvant ingredients and known additives that may be contained in health functional foods depending on the selection of those skilled in the art. Examples of the food that can be added may comprise meat, sausage, bread, chocolate, candy, snacks, confectionery, pizza, ramen, other noodles, gum, dairy products comprising ice cream, various soups, beverages, tea, drinks, alcoholic beverages, vitamin complexes, and the like, and the food may be prepared by adding an extract according to the present invention as a main component into squeezed juice, tea, jelly, juice, and the like.

All the matters described in compositions, treatment methods and uses of the present invention are applied equally as long as they are inconsistent with each other.

The present invention will be described in more detail through the following examples. However, these Examples are provided only to embody the contents of the present invention and the present invention is not limited thereto.

### <Example 1> Forced swimming test (FST) results depending on combined administration of cycloserine and pentoxifylline

In order to test an effect of reducing depression depending on administration of the composite composition of the present invention, mice were performed to a forced swimming test (FST). The FST, which is to measure a coping strategy for acute stress that inevitably occurs, is an animal behavioral experiment commonly used to measure antidepressant effects.

To confirm the effect of reducing depression by combined administration of D-cycloserine (DCS) and pentoxifylline (Pen), each group of mice was administered orally with the vehicle (DW 5 ml/kg), DCS (administered alone at 10 mg/kg), pentoxifylline (administered alone at 80 mg/kg) and DCS + pentoxifylline (DCS 10 mg/kg and pentoxifylline 10, 20, 80, 160 mg/kg combination treatment group). After administration, the forced swimming test (FST) that measures depression-like behavior was performed on the mice.

Specifically, each mice group (C57BL/6) was individually placed in a 3L Pyrex beaker (13 cm in diameter, 24 cm in height), and the beakers were filled with 26 °C of water up to a depth of 9 cm. All mice were forced to swim for 6 minutes, and immobility time was measured for the last 4 minutes of the experiment. The immobility time is defined as the RNR time taken while the mouse is floating without struggling or taking only the movement required for keeping the mouse's head above the water surface.

As a result, each group administered with single DCS or pentoxifylline alone did not show significantly different in immobility as compared to the control group administered with the vehicle, but a group that was subjected to FST in an hour after being administered with DCS and pentoxifylline in combination, showed a significant decrease in immobility (FIG. 1). In particular, the inventors have found that the antidepressant effect is maximized when the weight ratio of DCS and pentoxifylline in the combination treatment group is 1 : 1 to 1 : 12, specifically, 1 : 2 to 1 : 8.

### <Example 2> Tail suspension test (TST) results depending on combined administration of cycloserine and pentoxifylline

In order to test an effect of reducing depression depending on administration of the composite composition of the present invention, mice were subjected to a tail suspension test (TST). The TST, which is to measure a stress in a rodent animal model, is based on the behavior of an experimental animal facing immobile posture in the face of short-term, irreversible stress. In the technical field of the present invention, the TST is used to measure the effect of antidepressant.

To confirm the effect of reducing depression by combined administration of D-cycloserine (DCS) and pentoxifylline (Pen), DCS, Pen alone or a composite composition were administered to all groups of mice (C57BL6). Each group of mice was administered with the vehicle (DW 5 ml/kg), DCS (administered alone at 10 mg/kg), pentoxifylline (administered alone at 80 mg/kg), and DCS + pentoxifylline (DCS 10 mg/kg and pentoxifylline 10, 20, 80, 160 mg/kg combination treatment group). After 1 hour, the tail suspension test (TST) that measures depression-like behavior was performed on the mice.

Specifically, a tape of 7 cm in length was prepared and one end was attached to the tail of the mouse in each group, and the other end was attached to the upper rod of the prepared TST tool (60 cm in width, 11.5 cm in length, 55 cm in height) to hang the mouse. The experiment started as soon as the mouse was hung, and the immobility time was measured for 6 minutes after the start of the experiment. Immobility time is defined as the amount of time a mouse spends without floundering its legs. If, after making a major movement, the leg moves like a swing even though the leg was not moved, that time was included.

As a result, each group administered with DCS or Pen alone did not show statistical significance as compared to the control group administered with the vehicle, but a group that was subjected to TST in an hour after being administered with DCS and Pen in combination, showed a significant decrease in immobility (FIG. 2). In particular, following Example 1, the inventors have reconfirmed that the antidepressant effect is maximized when the weight ratio of DCS and pentoxifylline in the combination treatment group is 1 : 1 to 1 : 12, specifically, 1 : 2 to 1 : 8.

## Claims

1. A pharmaceutical composition for preventing or treating depression comprising:
(i) cycloserine or a pharmaceutically acceptable salt thereof; and
(ii) pentoxifylline or a pharmaceutically acceptable salt thereof.

2. The pharmaceutical composition of claim 1, wherein the cycloserine is D-cycloserine represented by Formula 1:

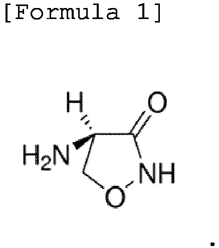

3. The pharmaceutical composition of claim 1, wherein a weight ratio of (i) the cycloserine or the pharmaceutically acceptable salt thereof; and (ii) the pentoxifylline or the pharmaceutically acceptable salt thereof is 1 : 1 to 1 : 12.

4. The pharmaceutical composition of claim 3, wherein a weight ratio of (i) the cycloserine or the pharmaceutically acceptable salt thereof; and (ii) the pentoxifylline or the pharmaceutically acceptable salt thereof is 1 : 2 to 1 : 8.

5. The pharmaceutical composition of claim 1, wherein the pharmaceutical composition is administered orally.

6. The pharmaceutical composition of claim 1, wherein (i) cycloserine or a pharmaceutically acceptable salt thereof; and (ii) pentoxifylline or a pharmaceutically acceptable salt thereof are administered simultaneously or at different times.

7. The pharmaceutical composition of claim 1, wherein the composition is a combination preparation comprising (i) cycloserine or a pharmaceutically acceptable salt thereof; and (ii) pentoxifylline or a pharmaceutically acceptable salt thereof.

8. A food composition for improving depression comprising:
(i) cycloserine or a pharmaceutically acceptable salt thereof; and
(ii) pentoxifylline or a pharmaceutically acceptable salt thereof.
